# EUROPEAN PATENT APPLICATION

(11) **EP 4 628 012 A1**
(43) Date of publication of application: **08.10.2025**
(21) Application number: 23912548.7
(22) Date of filing: 07.11.2023
(51) Int. Cl.: A61B 5/145, A61B 5/155, A61B 5/157

(54) **SENSOR APPLICATOR ASSEMBLY FOR BLOOD GLUCOSE METER**

(30) Priority: 30.12.2022 KR 20220190423
(71) Applicant: i-Sens, Inc., Seoul 06646 (KR)
(72) Inventor: CHOI, Won Seok, Seoul 06646 (KR); CHOI, Hyun Ho, Seoul 06646 (KR); CHO, Hee Gyung, Seoul 06646 (KR)
(74) Representative: BCKIP Part mbB
(86) International application number: PCT/KR2023/017769
(87) International publication number: WO 2024/143869

(57) **Abstract**

Provided is a sensor applicator assembly for a blood glucose monitoring including a body-attachable unit including an upper frame and a lower frame that is disposed under the upper frame and has a surface attached to a body and an applicator within which the body-attachable unit is disposed. The upper frame includes a first area and a second area disposed under the first area and in contact with the lower frame. A perimeter of an outer peripheral surface of the second area is identical to a perimeter of an outer peripheral surface of the lower frame.

## Description

### Technical Field

The present disclosure relates to a sensor applicator assembly for a blood glucose monitoring. More specifically, the present disclosure relates to a sensor applicator assembly for a blood glucose monitoring that may enable stable coupling and decoupling operations between an applicator and a body-attachable unit and easy manufacturing and component management of the body-attachable unit.

### Background Art

Diabetes is a chronic disease that considerably occurs to modern people. Diabetes occurs when an absolutely large amount of a sugar component is in blood because a balance between the blood and sugar is not redressed as insulin formed in pancreas is absolutely or relatively insufficient due to various causes such as obesity, stress, a poor diet, and innate heredity.

Generally, the blood contains a specific concentration of glucose, and a tissue cell gains energy therefrom.

However, when glucose increases beyond what the body needs, glucose cannot be properly stored in the liver, intestines, muscles, or fat cells, but is accumulated in the blood, leading a diabetic patient to maintain a significantly higher blood glucose level than a non-diabetic person. As excess blood glucose passes through tissues and is excreted in the urine, the sugar required for each body tissue becomes insufficient, causing abnormalities in each body tissue.

Diabetes has a feature that a subjective symptom thereof is not approximately obvious at an initial stage. As diabetes progresses, unique symptoms of diabetes, such as eating much, drinking much, polyuria, weight loss, general malaise, itching, and hand and foot injuries that are not cured and last long, appear. When diabetes further progresses, complications that develop into visual disturbance, high blood pressure, kidney disease, stroke, periodontal disease, a cramp, neuralgia, gangrene, and the like appear.

In order to diagnose diabetes and manage diabetes to prevent developing the complications, systematic measurement and treatment of the blood glucose may be done simultaneously.

For the diabetic patients and people having sugar measured to be more than normal in blood, multiple medical device manufacturers provide various types of blood glucose monitorings for measuring the blood glucose.

A scheme of a blood glucose monitoring includes a scheme of gathering blood from a fingertip of a user to perform blood glucose measurement on a one-time basis and a scheme of being attached to a stomach and an arm of the user to continuously perform the blood glucose measurement.

In a case of the diabetic patients, a hyperglycemia state and a hypoglycemia state generally alternate with each other. An emergency may occur in the hypoglycemia state, and the patient may lose consciousness or may die when the hypoglycemia state lasts long without supply of sugar. Thus, immediate detection of the hypoglycemia state is greatly important for the diabetic patients. However, accurate identification thereof is limited with a blood gathering-type blood glucose monitoring that intermittently measures the blood glucose.

Recently, in order to overcome such limitation, a continuous glucose monitoring (CGM) system that is inserted in a human body to measure a value of the blood glucose at intervals of several minutes is being developed. In order to minimize a pain or an aversion of the user, which is caused by blood gathering, the CGM system may insert a needle-shaped sensor into a region such as a stomach and an arm at which the pain is relatively less and then continuously measure the blood glucose.

The CGM system includes a body-attachable unit that is attached to a body of the user to measure blood glucose information and transmits the measured blood glucose information and a terminal that provides the blood glucose information received from the body-attachable unit to the user, allowing the user to monitor and manage the blood glucose information.

Here, the body-attachable unit includes a sensor module inserted in and attached to skin of the body of the user and measuring blood glucose from blood fluid of the user and a transmitter transmitting a level of the blood glucose measured by the sensor module to the terminal, and the sensor module is provided with a sensor probe formed in a needle shape to be inserted into subcutaneous fat and to extract the interstitial fluid (ISF).

An applicator is used to insert and attach the body-attachable unit to the body.

The applicator, as a configuration to insert and attach the body-attachable unit to the body, inserts and attaches the body-attachable unit to the body by the user operation, and by detaching and removing the applicator from the body in a state in which the body-attachable unit is inserted and attached to the body, the body-attachable unit is only allowed to remain attached to the body.

To implement the operations, the body-attachable unit is configured to be coupled to and decoupled from the applicator, and for the mutual coupling and decoupling between the body-attachable unit and the applicator, various elements, which are manufactured in different forms by each manufacturer, are used. A general body-attachable unit and an applicator according to a general technology are difficult to be manufactured due to complicated configurations thereof, and there is a problem in which the shapes and positions of various elements should be changed even for minor design modifications. Particularly, coupling or decoupling between the body-attachable unit and the applicator are not stably performed, causing pain during attachment of the body-attachable unit to the body or resulting in an unstable attachment of the body-attachable unit to the body.

### Detailed Description of the Invention

### Technical Goals

The present disclosure is to solve a problem of a general technology and to provide a sensor applicator assembly for a blood glucose monitoring that may enable stable coupling and decoupling operations between the applicator and a body-attachable unit, allow easy manufacturing of the body-attachable unit, enhance design flexibility, and simplify a form of a housing of the body-attachable unit to further expand and utilize an inner space.

### Technical solutions

Provided is a sensor applicator assembly for a blood glucose monitoring including a body-attachable unit including an upper frame and a lower frame that is disposed under the upper frame and has a surface attached to a body and an applicator within which the body-attachable unit is disposed. The upper frame may include a first area and a second area disposed under the first area and in contact with the lower frame. A perimeter of an outer peripheral surface of the second area may be identical to a perimeter of an outer peripheral surface of the lower frame.

Here, a side wall of the second area and a side wall of the lower frame may be flatly formed without step.

In addition, a maximum width of the second area may be identical to a maximum width of the lower frame.

In addition, a maximum width of both the upper frame and the lower frame may be a width of a cross-section perpendicular to a major axis of the body-attachable unit.

In addition, the sensor applicator assembly for the blood glucose monitoring may further include a plunger disposed within a main case of the applicator.

In addition, the plunger may be provided with a locking hook that is in contact with at least one area of the lower frame at a first position within the main case and released from contact at a second position positioned in a downward direction from the first position.

In addition, the sensor applicator assembly for the blood glucose monitoring may further include a friction member disposed on at least one of contact surfaces facing each other on which the locking hook is in contact with a housing including the upper frame and the lower frame.

In addition, the body-attachable unit may include a sensor member, one end portion of which is disposed within a housing including the upper frame and the lower frame, and another end portion of which protrudes outside the housing.

In addition, the plunger may include a sensor accommodating portion in which the body-attachable unit is inserted and accommodated. The locking hook may be coupled to an edge of the sensor accommodating portion and may be formed to be pivotable around a hinge axis in order for one end portion to be in contact with the housing under pressure or released from the contact.

In addition, a hook guide part may be formed on an inner surface of the main case to press the locking hook in a direction to be in contact with the housing or to release pressure depending on a position of the plunger.

In addition, a first elastic member may be disposed between the locking hook and the hook guide part to apply an elastic force to the locking hook in a direction in which the locking hook is in contact with the housing.

In addition, a second elastic member may be coupled between the locking hook and the plunger to apply an elastic force to the locking hook in a direction in which the locking hook is released from contact from the housing.

In addition, the elastic force of the first elastic member may be formed to be greater than the elastic force of the second elastic member.

### Effects of the Invention

According to the present disclosure, it is possible to easily perform manufacturing process of a body-attachable unit by enabling the body-attachable unit to be coupled to and decoupled from an applicator without forming a separate engaging coupling part in the body-attachable unit, enhance design flexibility for the body-attachable unit, and simplify a form of a housing of the body-attachable unit to further expand and utilize an inner space.

Furthermore, it is possible to further stabilize operational performance of the applicator and body attachment performance of the body-attachable unit by providing a more stable coupling and decoupling structure of the body-attachable unit and the applicator.

### Brief Description of Drawings

FIG. 1 is illustrating an example of usage of a blood glucose monitoring.
FIG. 2 is an exploded perspective view of an applicator for a blood glucose monitoring according to an example embodiment of the present disclosure.
FIG. 3 is a cross-sectional view illustrating a body-attachable unit installed in an applicator for a blood glucose monitoring according to an example embodiment of the present disclosure.
FIG. 4 is for illustrating an action of a shooting plate provided in an applicator for a blood glucose monitoring according to an example embodiment of the present disclosure.
FIGS. 5 and 6 are perspective views illustrating an assembly of a plunger of an applicator for a blood glucose monitoring and a needle separation unit according to an example embodiment of the present disclosure.
FIG. 7 is a cross-sectional view illustrating an assembly of a plunger of an applicator for a blood glucose monitoring and a needle separation unit according to an example embodiment of the present disclosure.
FIGS. 8 to 13 are illustrating a process of attaching a body-attachable unit to a body of a user by using an applicator for a blood glucose monitoring according to an example embodiment of the present disclosure.
FIG. 14 is a perspective view illustrating a coupling structure of a plunger and a body-attachable unit according to an example embodiment of the present disclosure.
FIG. 15 is a schematic perspective view illustrating a form of a hook guide part formed on an inner surface of a main case according to an example embodiment of the present disclosure.
FIG. 16 is a cross-sectional view illustrating a coupling structure of a plunger and a body-attachable unit at a first position within an applicator according to an example embodiment of the present disclosure.
FIG. 17 is a cross-sectional view illustrating a coupling structure of a plunger and a body-attachable unit at a second position within an applicator according to an example embodiment of the present disclosure.
FIG. 18 is an exemplary diagram illustrating an outer surface of a body-attachable unit according to an example embodiment of the present disclosure.
FIG. 19 is a perspective view illustrating a coupling structure of a plunger and a body-attachable unit according to an example embodiment of the present disclosure.
FIG. 20 is a cross-sectional view schematically illustrating a coupling structure of a plunger and a body-attachable unit illustrated in FIG. 19.

### Mode for Carrying Out the Invention

Hereinafter, a desired example embodiment of the present disclosure will be described in detail with reference to the accompanying drawings. To begin with, it should be noted that, for adding a reference numeral to an element in each drawing, the same elements have the same reference numeral if possible although illustrated in different drawings. Also, for description of the present disclosure, a detailed description of an associated element or function known to the public will be omitted when determined as obscuring the gist of the present disclosure.

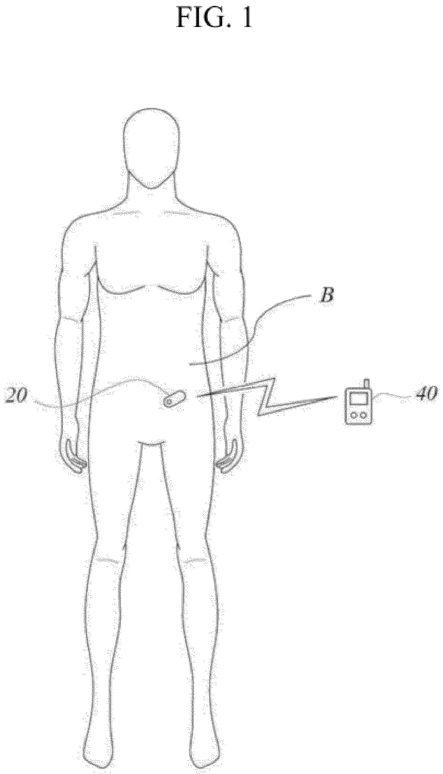

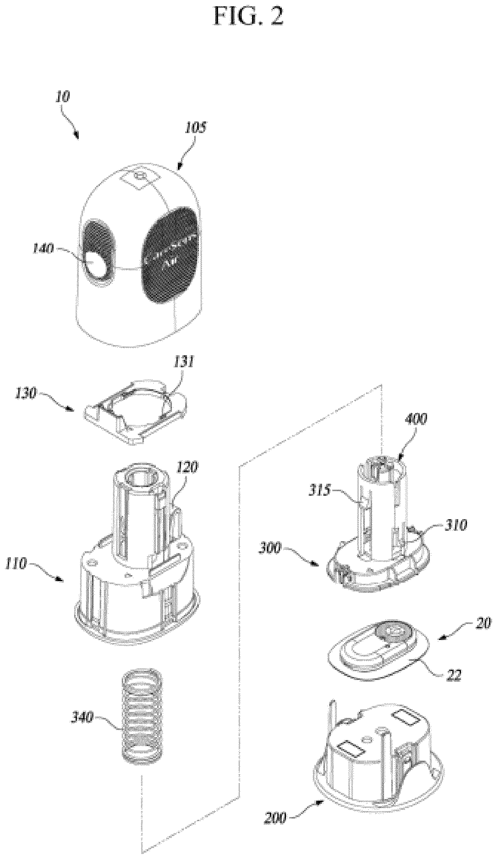

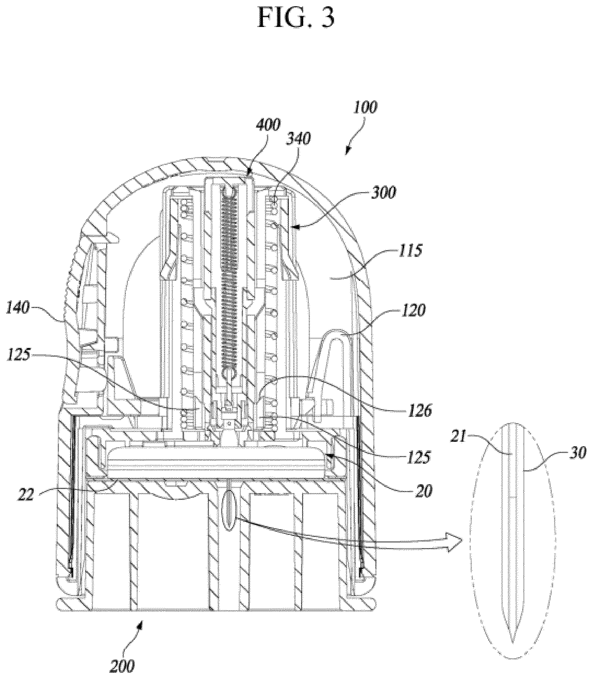

FIG. 1 is illustrating an example of usage of a blood glucose monitoring, and FIG. 2 is an exploded perspective view of an applicator for a blood glucose monitoring according to an example embodiment of the present disclosure, and FIG. 3 is a cross-sectional view illustrating a body-attachable unit installed in an applicator for a blood glucose monitoring according to an example embodiment of the present disclosure.

An applicator 10 for a blood glucose monitoring according to an example embodiment of the present disclosure is to attach a body-attachable unit 20 to measure blood glucose to a body B. A blood glucose monitoring to which the applicator 10 for the blood glucose monitoring according to an example embodiment of the present disclosure is applied may refer to a continuous glucose monitoring (CGM) system.

As shown in FIG. 1, the body-attachable unit 20 may be attached to the body B by the applicator 10 to periodically measure blood glucose from the body B and wirelessly transmit measured data to an external terminal 40. The body-attachable unit 20 may include a sensor member 21, a portion of which is inserted into the body, an adhesive tape 22 to attach to the body, a wireless communication chip that may wirelessly communicate with the external terminal 40, and a battery to measure blood glucose and wirelessly transmit measured data.

The applicator 10 according to an example embodiment of the present disclosure may have a structure in which the body-attachable unit 20 may be installed. The applicator 10 may eject the body-attachable unit 20 outside by being operated through user operation. The body-attachable unit 20 may be installed within the applicator 10 and attached to the body B by being ejected from the applicator 10 according to an operation of the applicator 10.

The applicator 10 according to an example embodiment of the present disclosure may be provided to a user in a state in which the body-attachable unit 20 is installed within, but the present disclosure is not limited thereto.

As the body-attachable unit 20 is provided with the adhesive tape 22 on one side, the body-attachable unit 20 may be attached to the body B of the user using the adhesive tape 22. The body-attachable unit 20 may be installed in the applicator 10 in a state in which the adhesive tape 22 is covered with a release liner. One portion of the release liner may be attached to a protective cap 200 to be removed in a process in which the protective cap 200 of the applicator 10 is separated from a main case 100.

The applicator 10 may have a structure to fix the body-attachable unit 20 in a state of being installed within and to release a holding force for the body-attachable unit 20 in a state in which the body-attachable unit 20 is ejected. In the state in which the body-attachable unit 20 is installed within the applicator 10, the body-attachable unit 20 may be maintained in a fixed state to the applicator 10. And after the body-attachable unit 20 is ejected from the applicator 10, the body-attachable unit 20 may remain on the body B of the user, being separated from the applicator 10.

In a process in which the body-attachable unit 20 is ejected from the applicator 10, a needle 30 may be coupled to be separable to the body-attachable unit 20 to allow the sensor member 21 to be stably inserted into the body B of the user. The needle 30 may be configured to surround one end portion of the sensor member 21 and to be inserted together with the sensor member 21 into the body, so that the one end portion of the sensor member 21 is stably inserted into the body B.

The needle 30 may be coupled to the body-attachable unit 20 to penetrate the body-attachable unit 20 in a thickness direction. One end of the needle 30 may be in a sharp form to penetrate skin of the body B of the user and to be smoothly inserted into the body B of the user, and on another end of the needle 30 is provided with a needle head 31. The needle 30 may play a role to support the sensor member 21 to be stably inserted into the body B by penetrating the skin of the body B of the user before the sensor member 21 when the body-attachable unit 20 is ejected from the applicator 10. After the body-attachable unit 20 is attached to the body B of the user, the needle 30 may be separated from the body B of the user by a needle separation unit 400 of the applicator 10.

Hereinafter, detailed configuration of the applicator 10 according to an example embodiment of the present disclosure is described in more detail.

The applicator 10 according to an example embodiment of the present disclosure may include the main case 100 where a pressing button 140 is installed on one side to be pressed and operated by the user, a plunger 300 supporting the body-attachable unit 20 and movably installed within the main case 100, a plunger spring 340 applying an elastic force to the plunger 300, and the needle separation unit 400 to separate the needle 30 that is coupled to the body-attachable unit 20 from the body-attachable unit 20.

The plunger 300 may eject the body-attachable unit 20 outside by moving from a first position P1 to a second position P2 within the main case 100. Here, the first position P1 may refer to an upper side within the main case 100, and the second position P2 may refer to a lower side within the main case 100. Thus, a direction moving from the first position P1 to the second position P2 may be defined as an external ejection direction. On one end portion of the main case 100, the protective cap 200 is coupled to be separable to protect the body-attachable unit 20 within the main case 100.

The protective cap 200 is coupled to the main case 100 not to expose the body-attachable unit 20 outside in a state in which the body-attachable unit 20 is coupled to the plunger 300. The user may attach the body-attachable unit 20 to the body by operating the applicator 10 only after separating the protective cap 200.

The protective cap 200 may not only protect the body-attachable unit 20 that is inserted within the main case 100 not to be exposed outside, but also perform a support function for the body-attachable unit 20. The protective cap 200 may improve overall structural stability of the applicator 10 by being coupled to the main case 100.

The main case 100 may include an outer case 105, on one side of which the pressing button 140 is installed, and an inner case 110 that is formed to guide a straight path of the plunger 300 by being coupled inside the outer case 105. Inside the main case 100, an accommodating space 115 may be formed to accommodate the plunger 300, the needle separation unit 400, and the body-attachable unit 20, and the like. The accommodating space 115 may be opened to the outside, and the body-attachable unit 20 may be ejected outside through the open portion of the accommodating space 115. The opened portion of the accommodating space 115 may be opened and closed by the protective cap 200.

The pressing button 140 may be pressed to be operated by the user. A shooting plate 130, which moves in response to a pressing operation of the pressing button 140, may be installed to be movable within the main case 100.

The shooting plate 130 may move while being seated in and supported by the inner case 110. The shooting plate 130 may slide to move in response to the pressing operation of the pressing button 140. The plunger 300 may be engaged with the shooting plate 130 at the first position P1 and disengaged from the shooting plate 130 as the shooting plate 130 moves. When disengaged from the shooting plate 130, the plunger 300 may move to the second position P2 by the elastic force of the plunger spring 340.

The pressing button 140 may be modified into other various structures that may operate the shooting plate 130 according to an operation of the user, in addition to the illustrated pressing operation manner.

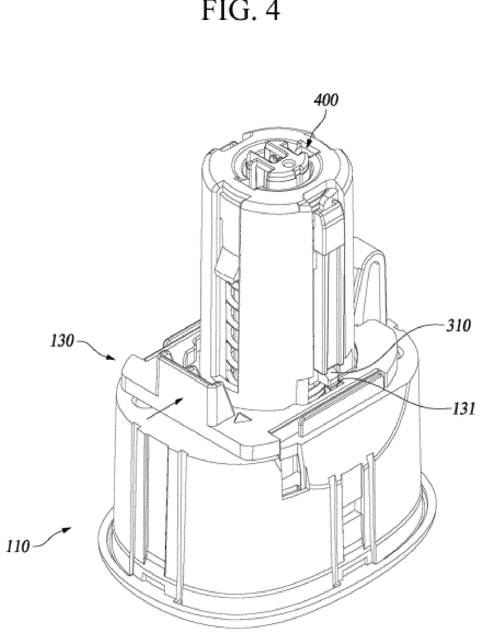

As shown in FIG. 4, the shooting plate 130 may be provided with a supporting part 131 on one side, with which an engaging hook 310 of the plunger 300 may be engaged. The plunger 300 may be fixed at the first position P1 as the engaging hook 310 is caught on the supporting part 131. The plunger 300 may move to the second position P2 when the engaging hook 310 is disengaged from the supporting part 131 as the shooting plate 130 is moved by the pressing button 140.

A specific form of the shooting plate 130 or the engaging structure of the shooting plate 130 and the plunger 300 is not limited to what is illustrated but may be modified in various ways.

The inner case 110 may be provided with a return member 120 that elastically supports the shooting plate 130. The return member 120 may apply an elastic force to the shooting plate 130 in the opposite direction to a pressing direction of the pressing button 140. The shooting plate 130 may maintain a stable engaging state with the engaging hook 310 of the plunger 300 by being supported by the return member 120.

The plunger 300 is installed within the main case 100 to support the body-attachable unit 20. The plunger 300 may eject the body-attachable unit 20 from the main case 100 by moving from the first position P1 to the second position P2 by the elastic force of the plunger spring 340. The plunger 300 may be provided with a plunger projection 315. The plunger projection 315 may limit a range of movement of the plunger 300. The movement of the plunger 300 may be limited in such a manner that the plunger projection 315 is engaged with one side of the inner case 110 when the plunger 300 moves to the second position P2. Accordingly, the plunger 300 may move only to the second position P2 and stop by the elastic force of the plunger spring 340 and not slip out of the main case 100.

A sensor accommodating portion 320 into which the body-attachable unit 20 is inserted may be formed on one end portion of the plunger 300. The body-attachable unit 20 may be installed in the sensor accommodating portion 320 and may move from the first position P1 to the second position P2 together with the plunger 300. When the plunger 300 and the body-attachable unit 20 move to the second position P2, the sensor member 21 of the body-attachable unit 20 may be inserted into the body B of the user.

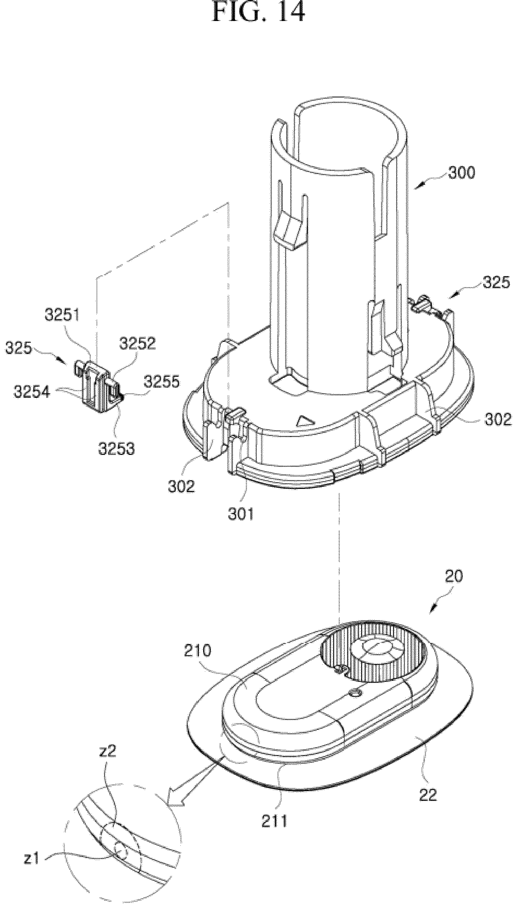

An edge of the sensor accommodating portion 320 may be provided with a locking hook 325 that fixes the body-attachable unit 20 by being engaged with an edge of the body-attachable unit 20 that is inserted into the sensor accommodating portion 320. The locking hook 325 may be coupled to be pivotable to the plunger 300 and may fix the body-attachable unit 20 by being engaged with an edge of the body-attachable unit 20 in a state in which the plunger 300 is positioned at the first position P1. In addition, the locking hook 325 may be disengaged from the body-attachable unit 20 in a process in which the user separates the applicator 10 from the body-attachable unit 20 that is attached to the body B of the user, in a state in which the plunger 300 is in the second position P2. A detailed description with regard to this will be described later mainly with reference to FIGS. 14 to 21.

In addition, a through hole 335 through which the needle 30 may pass may be formed in the plunger 300. The through hole 335 may be provided within the plunger 300 so as to connect to the sensor accommodating portion 320. The needle 30 may pass the through hole 335 and be coupled to the body-attachable unit 20 that is installed in the sensor accommodating portion 320.

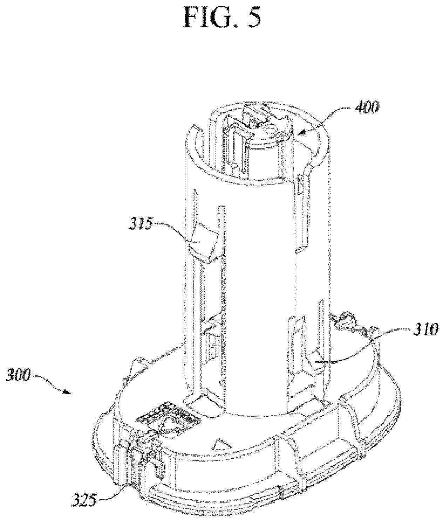

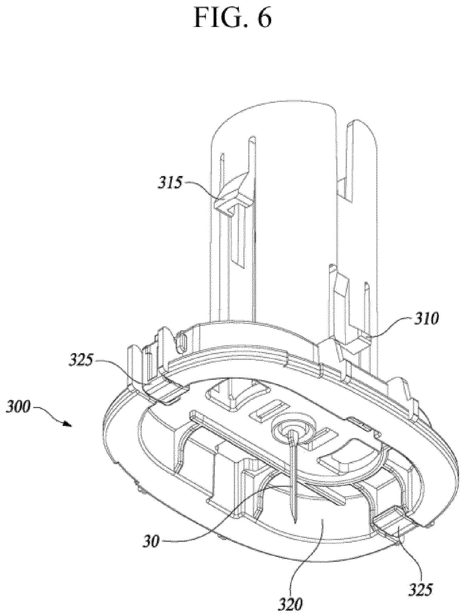

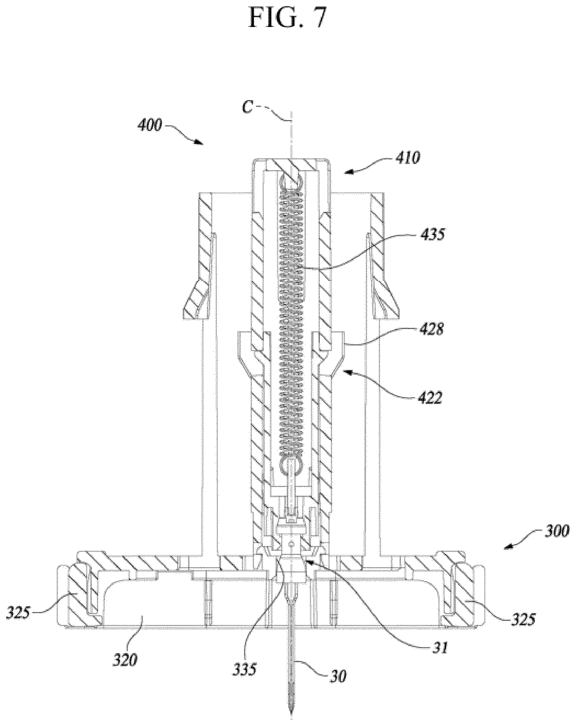

Referring to FIGS. 5 to 7, the needle separation unit 400 may separate the needle 30 that is coupled to the body-attachable unit 20 from the body B of the user as the body-attachable unit 20 moves from the first position P1 to the second position P2. The needle separation unit 400 may include a support column 410 that is fixed to the plunger 300, a needle holder 422 that is coupled to the needle head 31 by installed to be movable in the support column 410, and a needle separation spring 435 elastically supporting the needle holder 422.

The support column 410 may be fixed to the plunger 300 and may move from the first position P1 to the second position P2 together with the plunger 300, and the needle separation spring 435 may be installed to apply an elastic force to the needle holder 422 in a direction separating the needle holder 422 from the body-attachable unit 20, and the needle holder 422 may be fixed in a state of being engaged with the support column 410, and a trigger 428 may be formed on an outer surface to protrude outside the support column 410.

At this point, a pressing portion 125 that may be in contact with the trigger 428 under pressure may be formed in the inner case 110 while the support column 410 moves from the first position P1 to the second position P2 together with the plunger 300.

Accordingly, the trigger 428 may be pressed by the pressing portion 125 in a process where the plunger 300 moves to the second position according to an operation of the applicator, and accordingly, as the needle holder 422 is disengaged from the support column 410, the needle holder 422 and the needle 30 may move upward by the elastic force of the needle separation spring 435 and be separated from the body. In this state, when the applicator is separated and removed from the body-attachable unit 20, only the body-attachable unit 20 may be left attached to the body.

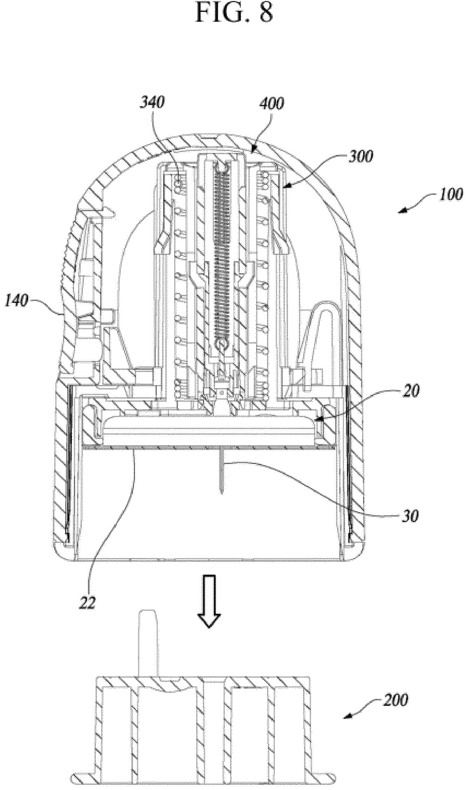

Next, a process of attaching the body-attachable unit 20 to the body B of the user by using the applicator 10 for the blood glucose monitoring according to an example embodiment of the present disclosure will be described with reference to FIGS. 8 to 13.

First, the protective cap 200 may be separated as illustrated in FIG. 8. In the process of separating the protective cap 200, the release liner of the body-attachable unit 20 may be separated together with the protective cap 200 to expose the adhesive tape 22 of the body-attachable unit 20.

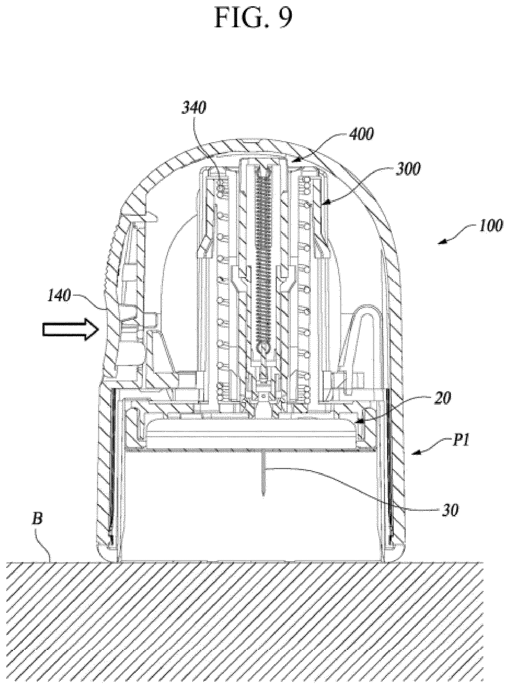

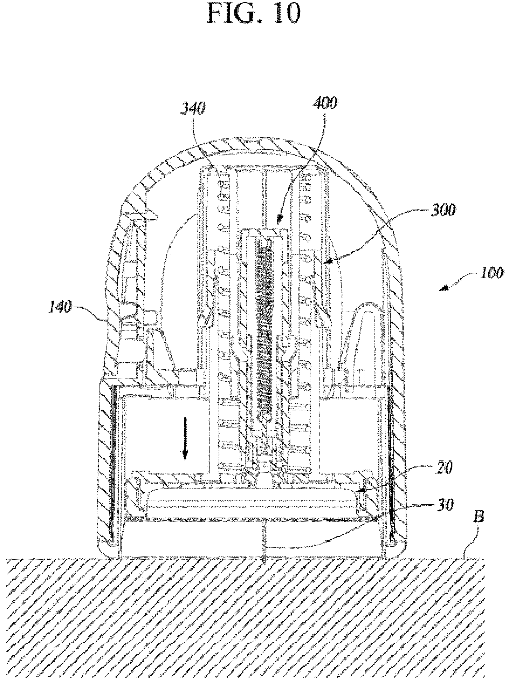

Subsequently, as illustrated in FIG. 9, the applicator 10 may be positioned on the body B of the user to whom the body-attachable unit 20 is attached, and the pressing button 140 may be pressed and operated. When the pressing button 140 is operated, the shooting plate 130 may move, and a holding force for the plunger 300 that is fixed at the first position P1 may be released. When the holding force for the plunger 300 is released by the shooting plate 130, as shown in FIG. 10, the plunger 300 that is provided with the body-attachable unit 20 may move to the second position P2 by the plunger spring 340, and the needle 30 and the sensor member 21 of the body-attachable unit 20 may penetrate the skin of the user and be inserted into the body B.

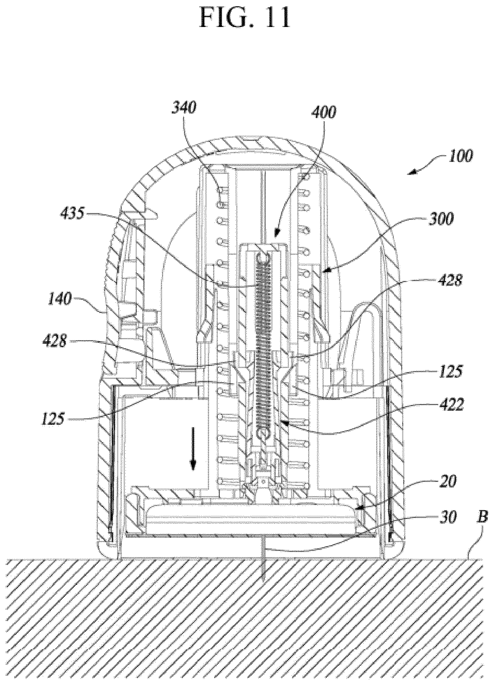

As illustrated in FIG. 11, when the trigger 428 of the needle separation unit 400 reaches the pressing portion 125 of the main case 100 while the plunger 300 moves to the second position P2, the trigger 428 may be pressed by the pressing portion 125 and may release a holding force for the needle holder 422. At the same time, the needle holder 422 may move in a direction away from the body B of the user by the elastic force of the needle separation spring 435.

As the needle holder 422 moves by the elastic force of the needle separation spring 435, the needle 30 that is inserted into the body B together with the sensor member 21 of the body-attachable unit 20 may be separated from the body-attachable unit 20 as being pulled by the needle holder 422.

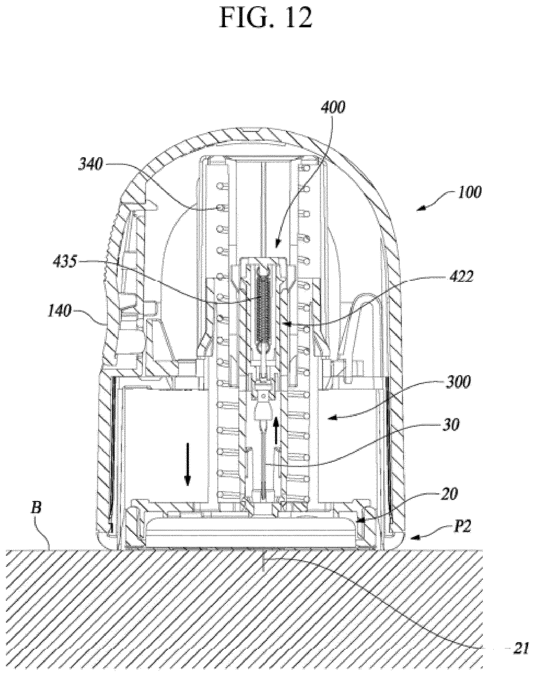

Continuously, as illustrated in FIG. 12, the plunger 300 may move to the second position P2 by the elastic force of the plunger spring 340 and stop. At this point, the body-attachable unit 20 may be attached to the body B of the user by the adhesive tape 22.

A point when the needle holder 422 separates the needle 30 from the body-attachable unit 20 while the body-attachable unit 20 moves to the second position P2 may be set to an appropriate point after guiding the needle 30 to penetrate the skin of the body B of the user and insert the sensor member 21 into the body B of the user. To this end, the pressing portion 125 may be disposed at an appropriate position in a moving path of the needle holder 422 depending on a length of the needle 30 and the like.

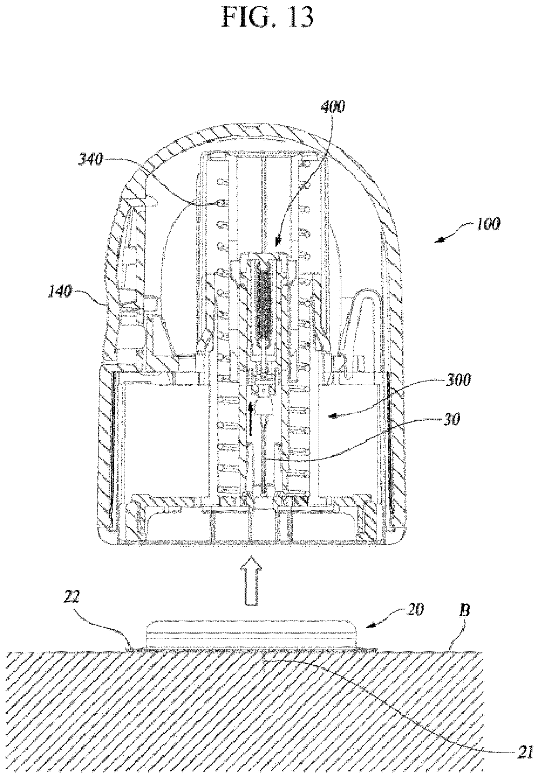

As illustrated in FIG. 13, after the body-attachable unit 20 is attached to the body B of the user, the attachment work for the body-attachable unit 20 may be completed when the applicator 10 is separated from the body-attachable unit 20.

Subsequently, the body-attachable unit 20 may measure blood glucose of the user and transmit measured information to the external terminal 40.

Next, a coupling structure of the plunger 300 and the body-attachable unit 20 is described in more detail mainly with reference to FIGS. 14 to 19.

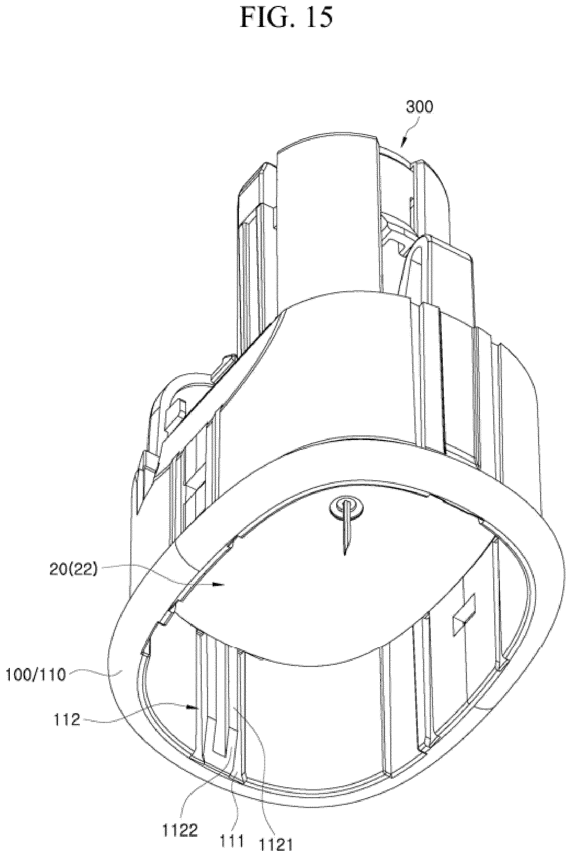

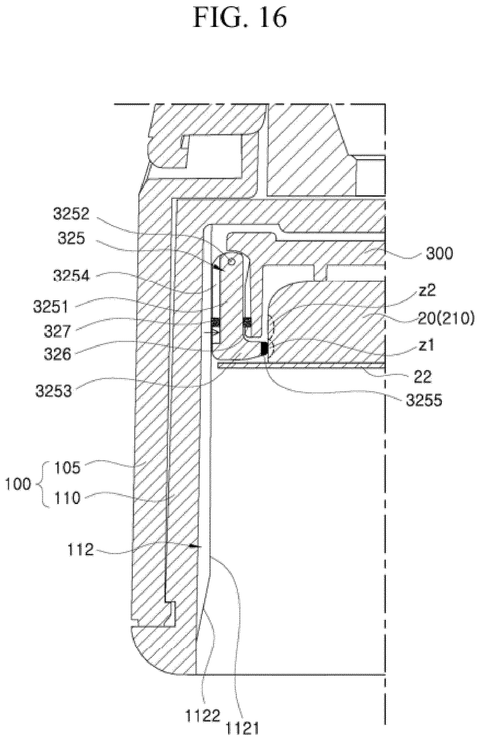

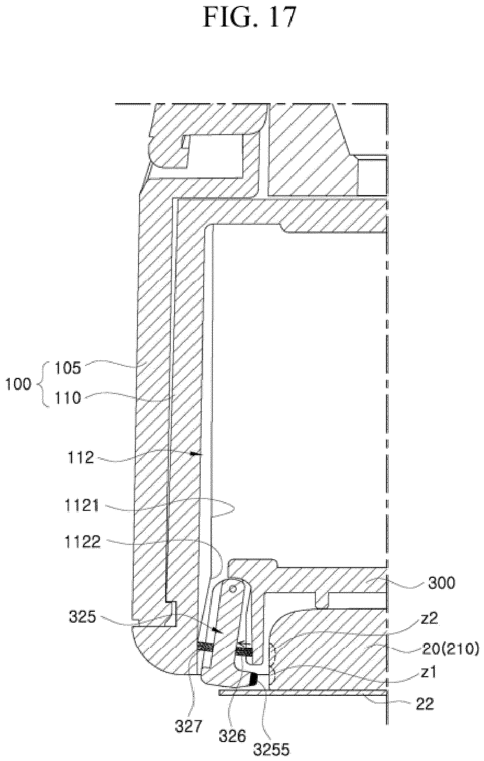

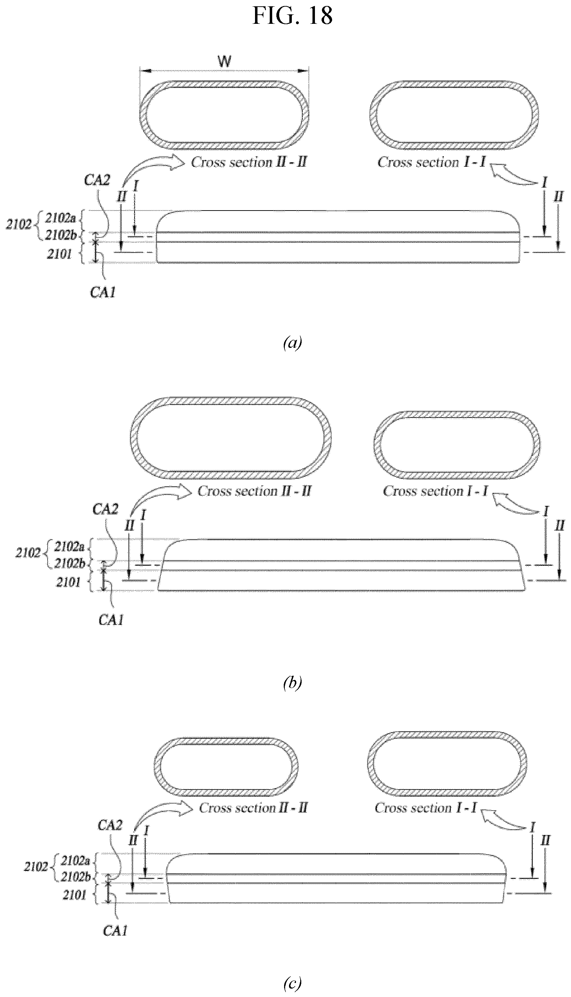

FIG. 14 is a perspective view illustrating a coupling structure of a plunger and a body-attachable unit according to an example embodiment of the present disclosure, and FIG. 15 is a schematic perspective view illustrating a form of a hook guide part formed on an inner surface of a main case according to an example embodiment of the present disclosure, and FIG. 16 is a cross-sectional view illustrating a coupling structure of a plunger and a body-attachable unit at a first position within an applicator according to an example embodiment of the present disclosure, and FIG. 17 is a cross-sectional view illustrating a coupling structure of a plunger and a body-attachable unit at a second position within an applicator according to an example embodiment of the present disclosure, and FIG. 18 is an exemplary diagram illustrating an outer surface of a body-attachable unit according to an example embodiment of the present disclosure.

As described above, the plunger 300 may move from the first position P1 to the second position P2 within the applicator 10, and the body-attachable unit 20, inserted and accommodated in the sensor accommodating portion 320 of the plunger 300, may move from the first position P1 to the second position P2 together with the plunger 300.

At this point, the plunger 300 may be coupled to the locking hook 325 that is coupled to the body-attachable unit 20 at the first position P1 and decoupled from the body-attachable unit 20 at the second position P2. At this point, on the body-attachable unit 20, an engaging coupling part corresponding to be coupled to the locking hook 325 may not be separately formed, and the locking hook 325 may be coupled to the body-attachable unit 20 in a form of being in contact under pressure with an outer surface of the body-attachable unit 20.

For example, the locking hook 325 may be formed to be in contact under pressure with both side surfaces facing each other of the body-attachable unit 20 at the first position P1, and to release the pressing contact from the both side surfaces at the second position P2.

In such a manner, the locking hook 325 may be installed in the plunger 300 to be coupled to and decoupled from the body-attachable unit 20, but as needed, the coupling structure of the body-attachable unit 20 and the plunger 300 may be modified in various ways, for example, with the body-attachable unit 20 simply inserted and coupled to the plunger 300 without the locking hook 325. Hereinafter, a structure in which the locking hook 325 is installed in the plunger 300 will be mainly described for convenience of description.

According to an example embodiment of the present disclosure, the engaging coupling part to be engaged with and coupled to the locking hook 325 may not be separately formed in the body-attachable unit 20. The engaging coupling part may be generally in a structure in which a step or a coupling groove is formed for engaging and coupling, but according to an example embodiment of the present disclosure, as the body-attachable unit 20 may not have the engaging coupling part such a concave groove, among outer surfaces of the body-attachable unit 20, a part that is in contact under pressure with the locking hook 325 may form a continuous surface with an adjacent part.

In other words, as the engaging coupling part such as a concave groove is not formed at a contact area Z1 that is in contact under pressure with the locking hook 325, a surface of the contact area Z1 may form a continuous surface with a surface of an adjacent area Z2 that is adjacent to the contact area Z1, without any discontinuity. For example, the surface of the contact area Z1 and the surface of the adjacent area Z2 may be formed to have an identical flat surface or a curved surface with an identical radius of curvature. Furthermore, the surface may have various forms including a surface that is continuously extended without any discontinuity in a boundary of a mutual surface, for example, with the surface forming a curved surface in which the curvature continuously changes. Here, a continuous surface may refer to a surface in which an incline of the surface changes continuously, not discontinuously.

The body-attachable unit 20 may include a housing 210 constituting an outer form and the sensor member 21 (see FIG. 3), one end of which protrudes downward outside the housing 210, and another end of which is installed within the housing 210, and the locking hook 325 may be formed to be in contact under pressure with an outer surface of the housing 210. Among outer surfaces of the housing 210, the surface of the contact area Z1, which is in contact with the locking hook 325, and the surface of the adjacent area Z2, which is adjacent to the contact area Z1, may be formed to be continuous with each other.

When examining the form of the body-attachable unit 20 in detail, as illustrated in FIG. 18, the housing 210 of the body-attachable unit 20 may include an upper frame 2102 and a lower frame 2101 disposed under the upper frame 2102 and having a surface attached to a body. The upper frame 2102 may include a first area 2102a having a separation surface such as various curved surfaces or flat surfaces and a second area 2102b that is positioned under the first area 2102a and in contact with the lower frame 2101.

At this point, a perimeter of an outer peripheral surface of the second area 2102b may be identically formed to a perimeter of an outer peripheral surface of the lower frame 2101. More specifically, a peripheral length and a form of an entire outer peripheral surface of the second area 2102b may be identically formed to a peripheral length and a form of an entire outer peripheral surface of the lower frame 2101. Here, identically formed may refer to being formed identically to each other within a margin of error that is predetermined in consideration of an allowable error that may occur in an entire production process such as a manufacturing and assembling process.

For example, as illustrated in FIG. 18 (a), an outer peripheral surface of the second area 2102b and an outer peripheral surface of the lower frame 2101 may be formed to have an identical vertical surface throughout an entire perimeter. In other words, the outer peripheral surface of the second area 2102b may have a vertical surface in an entire upper and lower area CA2, and the outer peripheral surface of the lower frame 2101 may have a vertical surface in an entire upper and lower area CA1, and the outer peripheral surface of the second area 2102b and the outer peripheral surface of the lower frame 2101 may be formed to have an identical surface (a vertical surface) without a step. In this case, a peripheral length and a form with regard to an outer peripheral surface of a horizontal cross section (cross section I-I) taken at a random height in the entire upper and lower area CA2 of the second area 2102b may be identical to a peripheral length and a form with regard to an outer peripheral surface of a horizontal cross section (cross section II-II) taken at a random height in the entire upper and lower area CA1 of the lower frame 2101. At this point, a maximum width W of the second area 2102b may be identically formed to a maximum width W of the lower frame 2101, and as illustrated in FIG. 18 (a), a maximum width W of the upper frame 2102 and a maximum width W of the lower frame 2101 may be configured as a maximum transverse length of a horizontal cross section. The maximum transverse length of the horizontal cross section may refer to a length in a widthwise direction. In other words, the maximum width W of the upper frame 2102 and the maximum width W of the lower frame 2101 may be configured as a widthwise length of a cross-sectional surface perpendicular to a major axis of the body-attachable unit 20. Here, the major axis of the body-attachable unit 20 may refer to a central axis of the body-attachable unit 20 that is disposed parallel to a direction in which the body-attachable unit 20 is ejected outside.

On the other hand, as illustrated in FIG. 18 (b), an outer peripheral surface of a second area 2102b and an outer peripheral surface of a lower frame 2101 may be formed to have an identical downward slope surface throughout an entire perimeter. In other words, the outer peripheral surface of the second area 2102b may have a downward slope surface in an entire upper and lower area CA2, and the outer peripheral surface of the lower frame 2101 may have a downward slope surface in an entire upper and lower area CA1, and the outer peripheral surface of the second area 2102b and the outer peripheral surface of the lower frame 2101 may be formed to have an identical surface (a downward slope surface) without a step. In this case, a peripheral shape with regard to an outer peripheral surface of a horizontal cross section (cross section I-I) taken at a random height in the entire upper and lower area CA2 of the second area 2102b may be identical to a peripheral shape with regard to an outer peripheral surface of a horizontal cross section (cross section II-II) taken at a random height in the entire upper and lower area CA1 of the lower frame 2101. However, as the outer peripheral surface of the second area 2102b and the outer peripheral surface of the lower frame 2101 form one downward slope surface, a perimeter of the outer peripheral surface of the horizontal cross section (cross section I-I) taken in the second area 2102b may be shorter than a perimeter of the outer peripheral surface of the horizontal cross section (cross section II-II) taken in the lower frame 2101. In other words, the outer peripheral surface of the second area 2102b and the outer peripheral surface of the lower frame 2101 may have different perimeter lengths but have identical shapes.

Furthermore, as illustrated in FIG. 18 (c), an outer peripheral surface of a second area 2102b and an outer peripheral surface of a lower frame 2101 may be formed to have an identical upward slope surface throughout an entire perimeter. In other words, the outer peripheral surface of the second area 2102b may have an upward slope surface in an entire upper and lower area CA2, and the outer peripheral surface of the lower frame 2101 may have an upward slope surface in an entire upper and lower area CA1, and the outer peripheral surface of the second area 2102b and the outer peripheral surface of the lower frame 2101 may be formed to have an identical surface (an upward slope surface) without a step. In this case, a peripheral shape with regard to an outer peripheral surface of a horizontal cross section (cross section I-I) taken at a random height in the entire upper and lower area CA2 of the second area 2102b may be identical to a peripheral shape with regard to an outer peripheral surface of a horizontal cross section (cross section II-II) taken at a random height in the entire upper and lower area CA1 of the lower frame 2101. However, as the outer peripheral surface of the second area 2102b and the outer peripheral surface of the lower frame 2101 form one upward slope surface, a perimeter of the outer peripheral surface of the horizontal cross section (cross section I-I) taken in the second area 2102b may be longer than a perimeter of the outer peripheral surface of the horizontal cross section (cross section II-II) taken in the lower frame 2101. In other words, in this case, the outer peripheral surface of the second area 2102b and the outer peripheral surface of the lower frame 2101 may also have different perimeter lengths but have identical shapes.

In such a manner, according to an example embodiment of the present disclosure, the perimeter of the outer peripheral surface of the horizontal cross section (cross section I-I) taken in the second area 2102b may have at least an identical peripheral shape, and an outline length of the perimeter may be identical or different, compared to the perimeter of the outer peripheral surface of the horizontal cross section (cross section II-II) taken in the lower frame 2101.

Accordingly, according to an example embodiment of the present disclosure, the outer surface of the housing 210 may be formed to have an identical continuous surface throughout an entire surface along a perimeter. To this end, a side wall of the second area 2102b may be flatly formed with a side wall of the lower frame 2101 without a step.

The locking hook 325 may be formed on an edge of the sensor accommodating portion 320 of the plunger 300 to be in contact under pressure with an outer surface of the body-attachable unit 20 that is inserted into the sensor accommodating portion 320 and to be coupled to and fix the body-attachable unit 20. The locking hook 325 may be coupled to be pivotable around a hinge axis 3252, and in a state in which the plunger 300 is in the first position P1, the locking hook 325 may be coupled to and may fix the body-attachable unit 20 by being pressed in an inward direction by the inner case 110, and in a state in which the plunger 300 is in the second position P2, the locking hook 325 may be released from pressure and decoupled from the body-attachable unit 20. In such a manner, to press and release pressure for the locking hook 325, a hook guide part 112 may be formed in the main case 100, more specifically, the inner case 110 of the main case 100.

The locking hook 325 may include, as illustrated in FIG. 14, a pivoting body 3251 on an upper portion of which the hinge axis 3252 is formed in a horizontal direction and a hook projection 3253 formed on a bottom of the pivoting body 3251 to protrude to be in contact under pressure with the outer surface of the body-attachable unit 20. On one surface (an opposite surface of the direction where the hook projection 3253 protrudes) of the pivoting body 3251, a pressing guide 3254 may be formed to protrude to enable a more stable pressure state by the hook guide part 112 of the inner case 110. The pressing guide 3254 may be formed to be long in an up and down direction on one surface of the pivoting body 3251.

A hinge axis coupling groove 301 may be formed on the plunger 300 to allow the hinge axis 3252 of the locking hook 325 to be coupled to be pivotable, and the hinge axis coupling groove 301 may be formed in a concavely dented shape on a top of a moving guide protruding part 302 that is formed on an outer surface of the plunger 300. The moving guide protruding part 302 may be formed on the outer surface of the plunger 300 to guide a moving path of the plunger 300 in a process in which the plunger 300 moves from the first position P1 to the second position P2, and in response to this, a guide groove 111 may be formed on an inner surface of the inner case 110 to guide the moving guide protruding part 302 of the plunger 300 to be inserted.

A plurality of locking hooks 325 may be coupled to the plunger 300, and in an example embodiment of the present disclosure, as illustrated in FIG. 14, two locking hooks 325 may be installed to face each other on both lateral end portions of the plunger 300. Of course, the form may be modified in various ways, for example, with four locking hooks 325 provided in a form where each pair faces each other. At this point, two locking hooks 325 paired with each other may be disposed to be symmetrically in contact under pressure with both side surfaces of the body-attachable unit 20.

In FIG. 15, a form of the hook guide part 112 formed on an inner surface of the inner case 110 of the main case 100. The hook guide part 112 may be formed to press the locking hook 325 in a direction of being in contact with the body-attachable unit 20 or release pressure, depending on a position of the plunger 300.

The hook guide part 112 may be formed to protrude in an inner central direction on an inner surface of the inner case 110 and formed to be long in an up and down direction. The hook guide part 112 may be formed to include a protruding surface 1121 and a concave surface 1122 on an inner surface, and the concave surface 1122 may be formed to be extended on a bottom of the protruding surface 1121 in a slope form where a protruding height decreases towards the bottom. On both sides of the hook guide part 112, the guide groove 111 may be formed to allow the moving guide protruding part 302 of the plunger 300 to be guided and inserted.

As illustrated in FIGS. 16 and 17, the protruding surface 1121 of the hook guide part 112 may be formed to press the locking hook 325, and the concave surface 1122 to release pressure for the locking hook 325. The protruding surface 1121 may be formed to press the locking hook 325 while the plunger 300 moves from the first position P1 to the second position P2, and more specifically, to press the pressing guide 3254, in contact with the pressing guide 3254 of the locking hook 325. The concave surface 1122 may be formed to release pressure for the locking hook 325 in a state in which the locking hook 325 is in the second position P2 together with the plunger 300. In other words, as the protruding surface 1121 protrudes relatively in an inward direction, the locking hook 325 may be pressed toward the engaging coupling part 211 in a process of the plunger 300 moving from the first position P1 to a section right before moving to the second position P2, and as the concave surface 1122 is relatively in a concave form, the locking hook 325 may be released from pressure in a state in which the plunger 300 is in the second position P2. At this point, the locking hook 325 may be in contact under pressure with the body-attachable unit 20 or released from the state of being in contact under pressure as pivoting around the hinge axis 3252 depending on a pressed state.

According to the structure, the locking hook 325 may be pressed in an inward direction by the protruding surface 1121 of the hook guide part 112 while the plunger 300 moves from the first position P1 to the second position P2 as illustrated in FIG. 16, and as pivoting around the hinge axis 3252, the locking hook 325 may be in contact under pressure with an outer surface of the body-attachable unit 20, which is inserted and accommodated in the sensor accommodating portion 301 of the plunger 300, and coupled to and fix the body-attachable unit 20.

As illustrated in FIG. 17, when the plunger 300 moves in a straight line to the second position P2, the locking hook 325 may be positioned at a position corresponding to the concave surface 1122 of the hook guide part 112. Accordingly, as the locking hook 325 may be released from pressure by the concave surface 1122 of the hook guide part 112, the locking hook 325 may pivot around the hinge axis 3252 and be released from the state of being in contact under pressure with the body-attachable unit 20. In this state, in other words, in a state in which the plunger 300 is in the second position P2 together with the body-attachable unit 20, the body-attachable unit 20 may be attached to the body by the adhesive tape 22 (see FIG. 12), and in this state, when the applicator 10 is separated and removed in a direction spaced apart from the body (see FIG. 13), as the locking hook 325 and the body-attachable unit 20 are released from being in contact under pressure and decoupled, only the applicator 10 may be separated and removed from the body, and only the body-attachable unit 20 may be attached to and remained on the body.

In such a manner, in an example embodiment of the present disclosure, even though the engaging coupling part is not separately formed in the body-attachable unit 20, a pressing contact force of the locking hook 325 may simply allow the body-attachable unit 20 to be coupled and fixed. Accordingly, in a process of manufacturing the body-attachable unit 20, as a separate molding and processing for engagement with the locking hook 325 are not needed, manufacture process may be easy, and design flexibility for the body-attachable unit 20 may be enhanced, and an inner space of the housing 210 of the body-attachable unit 20 may be further expanded to be used.

Meanwhile, the locking hook 325 may be pressed by the hook guide part 112 of the inner case 110 to allow the hook projection 3253 to be in contact under pressure with the outer surface of the housing 210 of the body-attachable unit 20 and to be coupled to and fix the body-attachable unit 20, and at this point, to enhance a coupling and fixing force of the locking hook 325 for the body-attachable unit 20, a friction member 3255 may be disposed on at least one of contact surfaces facing each other where the locking hook 325 and the body-attachable unit 20 are in contact with each other.

In FIGS. 14, 16, and 17, the friction member 3255 is illustrated to be formed on an end portion of the hook projection 3253 of the locking hook 325, but may alternatively be formed on the outer surface of the housing 210 of the body-attachable unit 20 or on both mutual contact surfaces of the locking hook 325 and the housing 210.

The friction member 3255 may be to enhance a mutual coupling force between the locking hook 325 and the body-attachable unit 20, and the friction member 3255 may be formed of a material with superior frictional force.

In addition, for an additional configuration to enhance the mutual coupling force between the locking hook 325 and the body-attachable unit 20, a first elastic member 327 may be separately provided.

The first elastic member 327, as illustrated in FIG. 16, may be disposed between the locking hook 325 and the hook guide part 112 to apply an elastic force to the locking hook 325 in a direction where the locking hook 325 is in contact with the housing 210 of the body-attachable unit 20. The first elastic member 327 may be formed of an elastic rubber material that is disposed between the locking hook 325 and an outer surface of the hook guide part 112, but may alternatively be formed in a form of a coil spring or a torsion spring coupled to the hinge axis 3252.

At this point, one end of the first elastic member 327 may be disposed to be pressed by the protruding surface 1121 of the hook guide part 112 and released from pressure by the concave surface 1122, and accordingly, while the plunger 300 moves from the first position to the second position, the first elastic member 327 may be pressed by the protruding surface 1121 and may apply an elastic force to the locking hook 325, and when the plunger 300 moves to the second position, the first elastic member 327 may be released from pressure by the concave surface 1122 and may not apply the elastic force to the locking hook 325.

The locking hook 325 may be in contact under pressure with the outer surface of the housing 210, pressed by the protruding surface 1121 of the hook guide part 112, but if the protruding surface 1121 of the hook guide part 112 may not have a uniform flat surface due to damage or a manufacturing defect, a pressing force of the locking hook 325 for the body-attachable unit 20 may decrease while the locking hook 325 passes through the defective section. In an example embodiment of the present disclosure, the first elastic member 327 may be separately and additionally provided so that the locking hook 325 continuously maintains the pressing force for the body-attachable unit 20 by the elastic force of the first elastic member 327. In other words, by the first elastic member 327 being provided, the locking hook 325 may continuously maintain the pressing force for the body-attachable unit 20 regardless of a defect of the hook guide part 112, and as additional an elastic pressing force is provided in addition to the pressing force by the hook guide part 112, the pressing contact force for the body-attachable unit 20 may be more enhanced.

Meanwhile, in a state in which the plunger 300 is in the second position P2, as the locking hook 325 may be positioned at a position corresponding to the concave surface 1122 of the hook guide part 112, technically, the locking hook 325 may only be released from pressure by the protruding surface 1121 and may not forcibly pivot in a direction to be released from the contact with the body-attachable unit 20.

In this case, as the locking hook 325 may freely pivot around the hinge axis 3252, in a process of separating and removing the applicator 10 from the body, in other words, in a process of the plunger 300 and the locking hook 325 positioned within the applicator 10 separated from the body-attachable unit 20, the state of being in contact under pressure between the locking hook 325 and the body-attachable unit 20 may not be maintained, and the coupling and fixing state may be released.

If the free pivoting state of the locking hook 325 is not properly operated due to an unknown reason, the state of being in contact under pressure between the locking hook 325 and the body-attachable unit 20 may not be smoothly released in a process of separating the applicator 10 from the body. In this case, as some of the force separating and removing the applicator 10 from the body may be transferred to the body-attachable unit 20, the attachment state of the body-attachable unit 20 to the body may become unstable, for example, with a portion of the adhesive tape 22 of the body-attachable unit 20 detached from the body.

Particularly, when the aforementioned first elastic member 327 is provided, as the locking hook 325 may be more likely to pivot in a direction to be in contact with the body-attachable unit 20, releasing the state of being in contact under pressure between the locking hook 325 and the body-attachable unit 20 may become more unstable.

In an example embodiment of the present disclosure, to solve the problem, a second elastic member 326 may be separately provided, as illustrated in FIGS. 16 and 17, to apply an elastic force to the locking hook 325 in a direction in which the locking hook 325 is released from being in contact under pressure with the body-attachable unit 20. The second elastic member 326 may be formed of an elastic rubber material disposed between the locking hook 325 and a wall of the plunger 300, but may alternatively be formed in a form of a coil spring or a torsion spring coupled to the hinge axis 3252.

As the locking hook 325 may be elastically supported by the second elastic member 326 in a direction to be released from being in contact under pressure with the body-attachable unit 20, the locking hook 325 may be forcibly released from being in contact under pressure with the body-attachable unit 20 by forcibly pivoting due to the elastic force of the second elastic member 326 when the plunger 300 moves to the second position P2. Accordingly, the state of the locking hook 325 decoupled from the body-attachable unit 20 may become more stable.

At this point, the elastic force of the first elastic member 327 may be formed to be greater than the elastic force of the second elastic member 326. Accordingly, as the elastic force of the first elastic member 327 is greater in a section in which the plunger 300 moves from the first position P1 to the second position P2, the locking hook 325 may be pressed toward the body-attachable unit 20 by the elastic force of the first elastic member 327, and when the plunger 300 moves to the second position P2, as the first elastic member 327 is released from pressing state by the concave surface 1122 of the hook guide part 112 and does not apply the elastic force, the locking hook 325 may pivot in a direction to be decoupled from the body-attachable unit 20 and the pressure may be released.

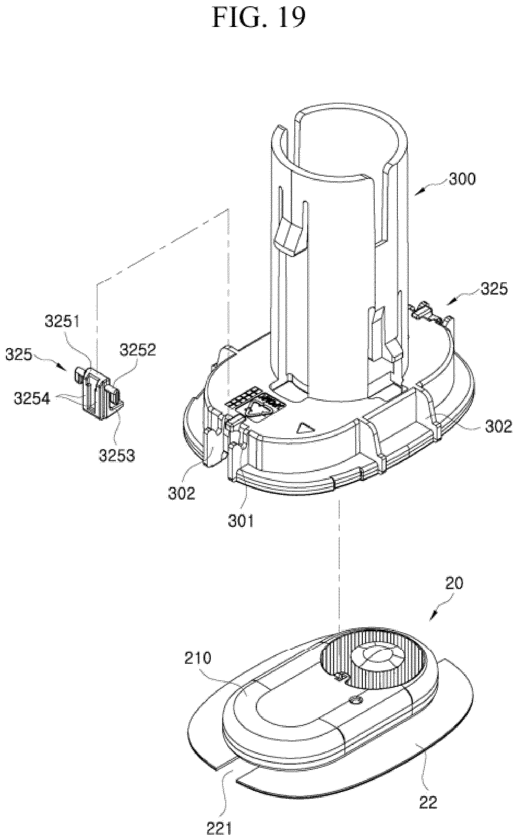

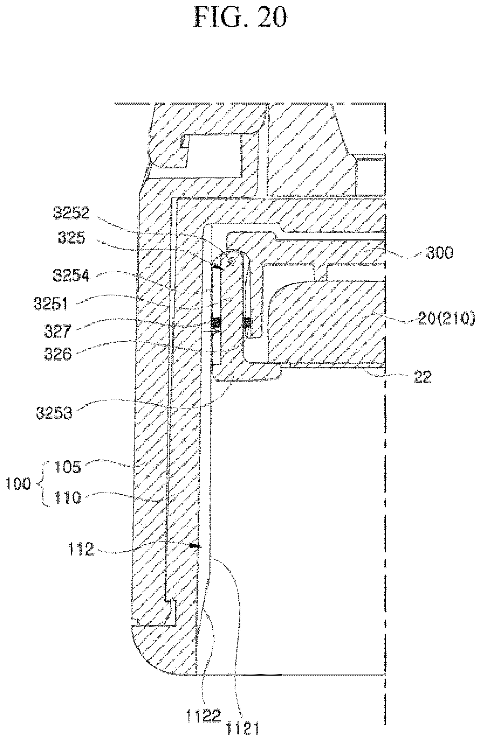

FIG. 19 is a perspective view illustrating a coupling structure of a plunger and a body-attachable unit according to an example embodiment of the present disclosure, and FIG. 20 is a cross-sectional view schematically illustrating a coupling structure of a plunger and a body-attachable unit illustrated in FIG. 19.

In FIGS. 14 to 18, the locking hook 325 is described to be coupled to and fix the body-attachable unit 20 by being in contact under pressure with the outer surface of the housing 210 of the body-attachable unit 20, but as illustrated in FIGS. 19 and 20, the locking hook 325 may be coupled to and fix the body-attachable unit 20 in a manner of engaged with and coupled to a bottom surface of the housing 210 of the body-attachable unit 20.

In this case, a slit part 221 in a slit form may be formed on a part of the adhesive tape 22 attached to a bottom surface of the housing 210 of the body-attachable unit 20, and the locking hook 325 may be in contact with the bottom surface of the housing 210 to be engaged and coupled through the slit part 221 area.

In such a manner, when the locking hook 325 is engaged with and coupled to the bottom surface of the housing 210 of the body-attachable unit 20, a more stable coupling force may be maintained compared to the structure in which an outer surface of the housing 210 is in contact under pressure.

The locking hook 325 may be, as illustrated in FIG. 20, engaged with and coupled to the bottom surface of the housing 210 by the protruding surface 1121 of the hook guide part 112 and the elastic force of the first elastic member 327 while the plunger 300 moves from the first position P1 to the second position P2. Even though not illustrated, when the plunger 300 moves to the second position P2, as described above, the locking hook 325 may pivot in a direction to be disengaged from the housing 210 by the elastic force of the second elastic member 326, thereby releasing from the state of being engaged with and coupled to the body-attachable unit 20.

In addition, as an operating structure with regard to engaging, coupling, and decoupling of the locking hook 325 is identical to the aforementioned structure, detailed description is omitted herein.

Meanwhile, even in this case, the outer surface of the housing 210 may be, as described in FIGS. 14 to 18, formed to have a continuous surface along an entire perimeter for ease of manufacture and space efficiency of the body-attachable unit 20. Of course, various forms having a discontinuous surface on the outer surface of the housing 210 may be added as needed by the user.

The above description is merely to describe an example of the technical idea of the present disclosure. Those skilled in the art to which the present disclosure belong may allow various changes and modifications within a range not deviating from the essential characteristics of the present disclosure. Thus, example embodiments disclosed in the present disclosure is to describe the technical idea of the present disclosure, not to limit the technical idea, and the range of the technical idea of the present disclosure is not limited to those example embodiments. The scope of the present disclosure should be construed with the following claims, and all technical ideas within a range equivalent thereto should be construed as being included in the scope of the present disclosure.

## Claims

1. A sensor applicator assembly for a blood glucose monitoring, comprising:
a body-attachable unit including an upper frame and a lower frame that is disposed under the upper frame and has a surface attached to a body; and an applicator within which the body-attachable unit is disposed,
wherein the upper frame includes a first area and a second area disposed under the first area and in contact with the lower frame, and
wherein a perimeter of an outer peripheral surface of the second area is identical to a perimeter of an outer peripheral surface of the lower frame.

2. The sensor applicator assembly for the blood glucose monitoring of claim 1,
wherein a side wall of the second area and a side wall of the lower frame are flatly formed without a step.

3. The sensor applicator assembly for the blood glucose monitoring of claim 1,
wherein a maximum width of the second area is identical to a maximum width of the lower frame.

4. The sensor applicator assembly for the blood glucose monitoring of claim 3,
wherein a maximum width of both the upper frame and the lower frame is a width of a cross-section perpendicular to a major axis of the body-attachable unit.

5. The sensor applicator assembly for the blood glucose monitoring of claim 1,
further comprising a plunger disposed within a main case of the applicator.

6. The sensor applicator assembly for the blood glucose monitoring of claim 5,
wherein the plunger
is provided with a locking hook that is in contact with at least one area of the lower frame at a first position within the main case and released from contact at a second position positioned in a downward direction from the first position.

7. The sensor applicator assembly for the blood glucose monitoring of claim 6,
further comprising a friction member disposed on at least one of contact surfaces facing each other on which the locking hook is in contact with a housing including the upper frame and the lower frame.

8. The sensor applicator assembly for the blood glucose monitoring of claim 1,
wherein the body-attachable unit
includes a sensor member, one end portion of which is disposed within a housing including the upper frame and the lower frame, and another end portion of which protrudes outside the housing.

9. The sensor applicator assembly for the blood glucose monitoring of claim 6,
wherein the plunger
includes a sensor accommodating portion in which the body-attachable unit is inserted and accommodated, and
wherein the locking hook
is coupled to an edge of the sensor accommodating portion and is formed to be pivotable around a hinge axis in order for one end portion to be in contact with the housing under pressure or released from contact.

10. The sensor applicator assembly for the blood glucose monitoring of claim 6,
wherein a hook guide part is formed on an inner surface of the main case to press the locking hook in a direction to be in contact with the housing or to release pressure depending on a position of the plunger.

11. The sensor applicator assembly for the blood glucose monitoring of claim 6,
wherein a first elastic member is disposed between the locking hook and the hook guide part to apply an elastic force to the locking hook in a direction in which the locking hook is in contact with the housing.

12. The sensor applicator assembly for the blood glucose monitoring of claim 11,
wherein a second elastic member is coupled between the locking hook and the plunger to apply an elastic force to the locking hook in a direction in which the locking hook is released from contact from the housing.

13. The sensor applicator assembly for the blood glucose monitoring of claim 12,
wherein the elastic force of the first elastic member is formed to be greater than the elastic force of the second elastic member.
